# NEW EUROPEAN PATENT SPECIFICATION

(11) **EP 0 166 611 B2**
(45) Date of publication and mention of the opposition decision: **10.03.1999**
(45) Mention of the grant of the patent: 16.08.1989
(21) Application number: 85304557.3
(22) Date of filing: 26.06.1985
(51) Int. Cl.: C07D 275/02, A01N 43/80, A01N 25/22, A61K 7/00

(54) **Stabilization of aqueous solutions of 5-chloro-3-isothiazolones**
Stabilisierung von wässrigen 5-Chloro-3-isothiazolonlösungen
Stabilisation de solutions aqueuses de 5-chloro-3-isothiazolones

(30) Priority: 27.06.1984 GB 8416314; 03.06.1985 US 740375
(43) Date of publication of application: 02.01.1986
(73) Proprietor: ROHM AND HAAS COMPANY, Philadelphia Pennsylvania 19105 (US)
(72) Inventor: Amick, David Richard, Chalfont, Pa. 18914 (US); Melamed, Sidney, Elkins Park, Pa. 19117 (US); Weiler, Ernest Deiter, Ambler, Pa. 19002 (US)
(74) Representative: Angell, David Whilton

(56) References cited:
- GB-A- 2 004 747
- US-A- 3 870 795
- US-A- 4 241 214

## Description

This invention is concerned with the use of hydroxy solvents to stabilize solutions of 5-chloro-3-isothiazolones.

Isothiazolones useful in the present invention are represented by the following structural formula: 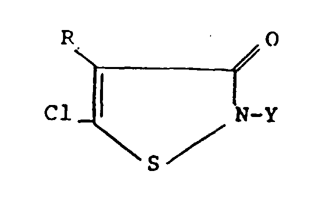 as defined below.

Representative Y substituents include methyl, ethyl, propyl, isopropyl, butyl, hexyl, octyl, cyclohexyl, benzyl, 3,4-dichlorobenzyl, 4-methoxybenzyl, 4-chlorobenzyl, 3,4-dichlorophenyl, 4-methoxyphenyl, hydroxymethyl, chloromethyl and chloropropyl.

In many instances it is desirable to minimize water, salt, and nitrate levels in isothiazolone biocides. For example, certain emulsions or dispersions require biocidal protection and are sensitive to shock resulting in a precipitate when salts, especially those containing divalent ions, are added. This precipitation precludes the use of biocides containing appreciable salt levels, especially in situations where mechanical stirring is not feasible.

In oils and fuels it is desirable to minimuze water and salt content. Water in contact with the organic matter in fuel creates conditions suitable for biological growth and the formation of sludge. Also, salts in oils and fuel result in ignition deposits which lead to clogging and corrosion of various mechanical components.

In some cosmetic formulations, it is important to control water and salt content. Eliminating the presence of nitrate salts avoids the possibility of nitrosamine formation. Nitrosarnines are suspected carcinogens.

This invention is directed to the reduction of water and salts, especially nitrates, in stable biocidal isothiazolone compositions.

This invention provides the use of 68 to 98.85% by weight of one or more hydroxy solvents of the formulae: 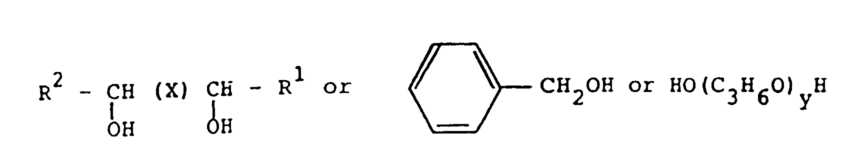 wherein R² and R¹ are hydrogen or lower alkyl, X is -CH₂OCH₂-, (CH₂)ₙ wherein n is an integer of 1 to 4 and y is an integer of 1 to 150; as a complete replacement for nitrate stabilisation salt in a stabilised solution of an isothiazolone which comprises 0.5 - 7% of the isothiazolone of the formula 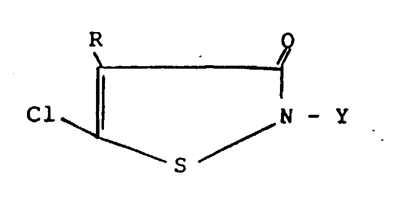 wherein
Y is an alkyl group of 1 to 8 carbon atoms optionally substituted with one or more hydroxy, halo, cyano, alkylamino, dialkylamino, arylamino, carboxy, carbalkoxy, alkoxy, aryloxy, alkylthio, arylthio, isothiazolonyl, haloalkoxy, carbamoxy, morpholino, piperidino or pyrrolidino groups, an unsubstituted or halo-substituted alkenyl or alkynyl of 2 to 8 carbon atoms, an unsubstituted cycloalkyl of 5 to 8 carbon atoms, an aralkyl group, optionally substituted with one or more halo, nitro,
   (C₁-C₄)alkyl or (C₁-C₄) alkoxy groups, or an aryl group optionally substituted with one or more halo, cyano, nitro, (C₁-C₄) alkyl, (C₁-C₄,) alkoxy C₁-C₄ alkyl-acylamino, (C₁-C₄) carbalkoxy or sulfamyl groups;
R is hydrogen, halo, or (C₁-C₄) alkyl; 0.5 to 14% water; from 0.15 to 7% of neutralization salt and no stabilisation salt, the percentages being by weight of the four components.

The stabilizing solvent used in this invention comprises one or more of those of the following structural formulae: 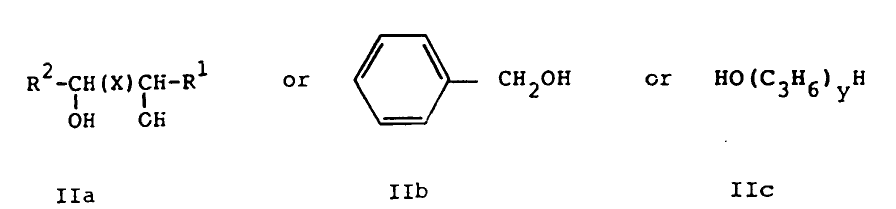 wherein R² and R¹ are hydrogen or (C₁-C₆) alkyl, such as methyl, ethyl, propyl, butyl and pentyl and X is -CH₂OCH₂- or -(CH₂)ₙ- where n is an integer of 1 to 4 and y is an integer of from 1 to 150.

By "lower" in connection with alkyl groups in this specification we mean "C₁-C₆".

Preferred stabilizing solvents include propylene glycol, benzyl alcohol, dipropylene glycol, polypropylene glycol and 1,5-pentanediol.

The stabilizing solvent is employed in an amount of from 68 to 98.85% by weight of the four specified components in the composition with a preferred effective amount being from about 92 to about 98% by weight.

Prior to this discovery it was known to employ organic solvents with metal nitrates (See especially U.S. Patent 3,870,795, Col. 3, lines 39-54). However, the solvents were not known to be useful as stabilizers. U.S. Patent 3,870,795, Col. 4, lines 35-45 discloses that nonaqueous solutions containing about 15% of 5-chloro-2-methyl-3-isothiazolone/2-methyl-3-isothiazolone (93:7) in dipropylene glycol completely decomposed in 28 days at 50°C.

U.S. Patents 4,241,214 and 4,396,413 describe metal salt complexes of isothiazolones of Formula I and their use as effective biocidal agents. Current commercial products containing these isothiazolones are sold as aqueous solutions containing divalent nitrate salts which serve as stabilizers for isothiazolones which would otherwise decompose upon storage.

While this invention may permit the stabilization of isothiazolones without employing stabilization salts, it should be recognized that this invention may also be used to permit the use of much lower concentrations of the stabilization salts than have hitherto been conventional. Useful stabilization salts which can be employed are disclosed in U.S. Patents 3,870,795 and 4,067,878. Preferred stabilization salts fall into two groups:
1) Metal nitrates, where the metal is barium, cadmium, calcium, chromium, cobalt, copper, iron, lead, lithium, magnesium, manganese, mercury, nickel, sodium, silver, strontium, tin, zinc and the like; and
2) Copper (2+) salts where the anion is halide, sulfate, nitrate, nitrite, acetate, chlorate, perchlorate, bisulfate, bicarbonate, oxalate, maleate, carbonate, or phosphate.

In the preparation of the 5-chloro-3-isothiazolones, it is desired to partially neutralize the intermediate isothiazolone hydrochloride salt to obtain a more stable product; this results in the formation of the "neutralization salt" as a by-product. The neutralization salt does not improve stability (except in the case where it is CuCl₂) and should not generally be considered a stabilizing salt.

Typical ranges for the content of the components in the compositions are illustrated in the following Tables (all percentages are parts by weight of the total of the four stated components). These ranges may be used individually or in any mathematically possible combination to give preferred formulations.

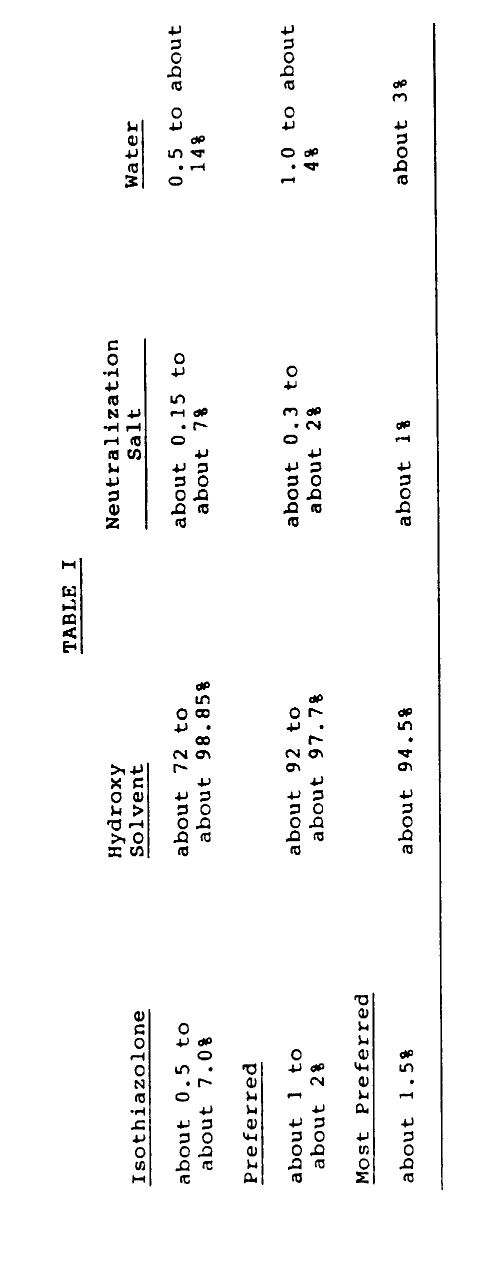

The following Table II illustrates ranges for the components in compositions of this invention when nitrates are employed. These ranges may be used individually or in any mathematically possible combination go give preferred formulations - and the percentages are by weight of the five stated components.

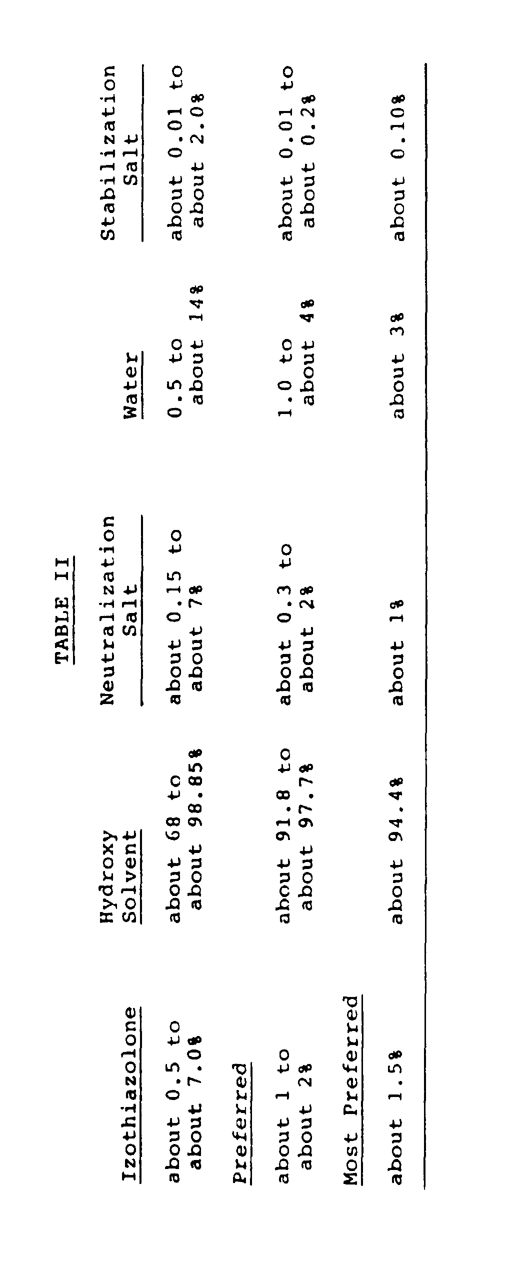

Especially preferred are those formulations which employ sodium chloride or copper chloride as the neutralizing salt, and no post-added stabilization salt.

Solutions of isothiazolones are used, inter alia, as watercooling system microbiocides, as preservatives for aqueous dispersions or organic polymers, as wood pulp white water slimicides, as cosmetic preservatives, as cutting oil, jet fuel and heating oil preservatives. Solutions of isothiazolones are also applied to solid substrates, such as of fabric, leather, or wood, as a preservative.

The products are especially useful as preservatives for the following: 1. Cosmetics, as the invention can be used to eliminate or substatially reduce the presence of nitrates which under certain conditions in the presence of amines or amine precursors may lead to the formation of nitrosoamines; 2. Oils and fuels, since added salts and moisture can be eliminated or minimized thus preventing potential corrosion, deposition or sludge formation; 3. Emulsions and dispersions that are sensitive to the addition of salts. Examples of these emulsions and dispersions are those contained in a wide variety of products, such as paints, cosmetics, floor polishes and binders.

The following Examples will further illustrate this invention. Furthermore they include comparative data to illustrate the improvement achievable by the invention. All parts and percentages are by weight and all temperatures in degrees Centigrade, unless otherwise stated.

In interpreting the results in the Examples it should be noted that our studies show that a temperature of 50°C causes an acceleration effect so that one week at that temperature is equivalent to 2 months at ordinary ambient temperature; two weeks is equivalent to 4 months; three weeks is equivalent to 6 months; and four weeks is equivalent to 8 months, and so on. A temperature of 65°C causes an acceleration effect so that one week at that temperature is equivalent to 7 months at ordinary ambient temperature; two weeks is equivalent to 14 months; three weeks is equivalent to 21 months; four weeks is equivalent to 28 months, and so on. We consider that any product which does not show signs of decomposition in one year (6 weeks at 50°C or about 2 weeks at 65°C) should be regarded as a stable product.

### Example 1

### Hair Shampoo - Nitrate and Nitrate free

A solution containing 3% active isothiazolones N-methyl-5-chloroisothiazolin-3-one and N-methylisothiazolin-3-one, 2% NaCI, 3% water in dipropylene glycol is used as a preservative for a hair shampoo. The biocide solution will contain no nitrate, nitrite or nitrosamines. The shampoo is treated with biocide solution to provide 20 ppm of active biocide. The resultant shampoo contains no nitrosamine and has no nitrate to react with amines in the shampoo composition.

### Example 2

### Stability (by HPLC) of 5-chloro-2-methylisothiazolin-3-one in Organic Solvents (90%) with 10% Water at 65°C (1% Al to start)

| Solvent | 1week | 2weeks | 3weeks | 4weeks |
|---|---|---|---|---|
| Dipropylene glycol | P | P | P | F |
| Propylene glycol | P | F | | |
| Ethylene glycol | P | F | | |
| Diethylene glycol | P | P/F | F | |
| Triethylene glycol | P/F | F | | |
| 1,5-Pentanediol | P | P | P/F | F |
| 2,4-Pentanediol | P | F | | |
| Benzyl alcohol | P | P | P | P |
| Triethylene glcyol, | F | | | |
| dimethyl ether | | | | |
| Acetonylacetone | P/F | F | | |
| Dimethylsulfoxide | F | | | |

This test compares the stabilizing capacity of various solvents in the presence of water.
Al (Active Ingredient) determined by HPLC (HPLC = High Pressure Liquid Chromatography)
P = essentially no loss of Al
F = Al totally decomposed
P/F = 20-80% of Al decomposed (by HPLC)

### Example 3

### Stability (by HPLC) of 5-chloro-2-methylisothiazolin-3-one in dipropylene glycol (DPG)/H2O at 65°C

| Test No | Initial % Isothiazolone | % DPG/H2O | % Salts | % lost | | | |
|---|---|---|---|---|---|---|---|
| | | | | 3 days | 1wk | 2wk | 4wk |
| 1 | 1 | 0/100 | 0 | 100 | -- | -- | -- |
| 2 | 1 | 92/8 | 0 | 0 | 0 | 5 | 100 |
| 3 | 1 | 92/8 | 1.5 Mg(NO₃)₂ | 0 | 0 | 0 | 2 |
| 4 | 10 | 92/8 | 1.5 Mg(NO₃)₂ | NA | 7 | 17 | 100 |
| | | | 1.0 MgCl₂ | | | | |
| 5 | 10 | 0/100 | 1.5 Mg(NO₃)₂ | 100 | -- | -- | -- |
| | | | 1.0 MgCl₂ | | | | |
| NA - Not analyzed at 3 days | | | | | | | |

This test illustrates how increasing water content lowers stability (1 vs 2 and 4 vs 5). Also illustrates increase stability using small amounts of a nitrate stabilizer (2 vs 3 and 4 vs 5).

### Example 4

### Preparation of 5.4% A.l. Formulation

To a 250-ml beaker was added 20 g of Kathon HCI salt¹ which had been powdered via mortar and pestle, and 25 g of water. Solid magnesium oxide (1.9 g) was dissolved in the mixture to give a two-phase solution having pH = 2.87. The toluene layer was removed (via separatory funnel), and 0.64 g of Cu(NO₃)₂. 3 H₂O (stabilizing salt) was dissolved in 23.5 g of the aqueous solution, and then diluted with 77.5 g of DPG to give pH = 2.1, Al = 5.4%, and Cu(NO₃)₂ level of about 0.5%, and MgCl₂ (neutralizing salt) level of about 2.4%.
¹ Kathon® biocide is a commercial product presently sold in a 15% mixture of N-methyl-5-chloroisothiazolin-3-one (A) and N-methylisothiazolin-3-one (B) is an approximate 80:20 ratio.

This formulation showed no loss in Al during heat aging for 5 wks/55°C or 4 wks/65°C.

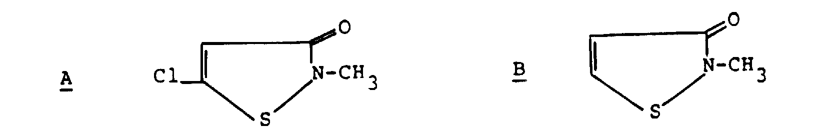

The product contains 60% water, 9% MgCl₂ and 15% Mg(NO₃)₂. A specially prepared version of Kathon for cosmetic applications contains 1.5% of an 80:20 mixture of A and B, 75% water, 1% MgCl₂, and 23% Mg(NO₃)₂. The high levels of Mg(NO₃)₂ are useful in stabilizing A in its aqueous environment, as demonstrated in U.S. Patents 3,870,795 and 4,067,878. BY using this invention, Mg(NO₃)₂ is no longer needed for stabilization. The isothiazolones can be isolated and combined with an equivalent of HCI to form the salt, i.e., "Kathon HCl".

### Example 5

### Thermal Stability Test

Thermal stability tests were run on a mixture of 1.13% of 5-chloro-2-methyl-isothiazolin-3-one and 0.37% of N-methyl-isothiazolin-3-one (= 1.5%) solution in propylene glycol, and on 0.7% and 1.5% active ingredient solutions in dipropylene glycol. These solutions were prepared by diluting KATHON biocide (see footnote of Example 4) to 1.5% active ingredient with the indicated organic solvents (w/w).

One hundred gram aliquots of each of the three solutions were placed in a 50°C hot air oven and stored for 3 months at 50°C. Five gram aliquots were removed from each of the three samples just prior to their placement in the oven, and again after 12 days, 33 days and three months at 50°C. These 5 gram aliquots were analyzed by HPLC for active ingredient content at the time they were removed from the stored samples.

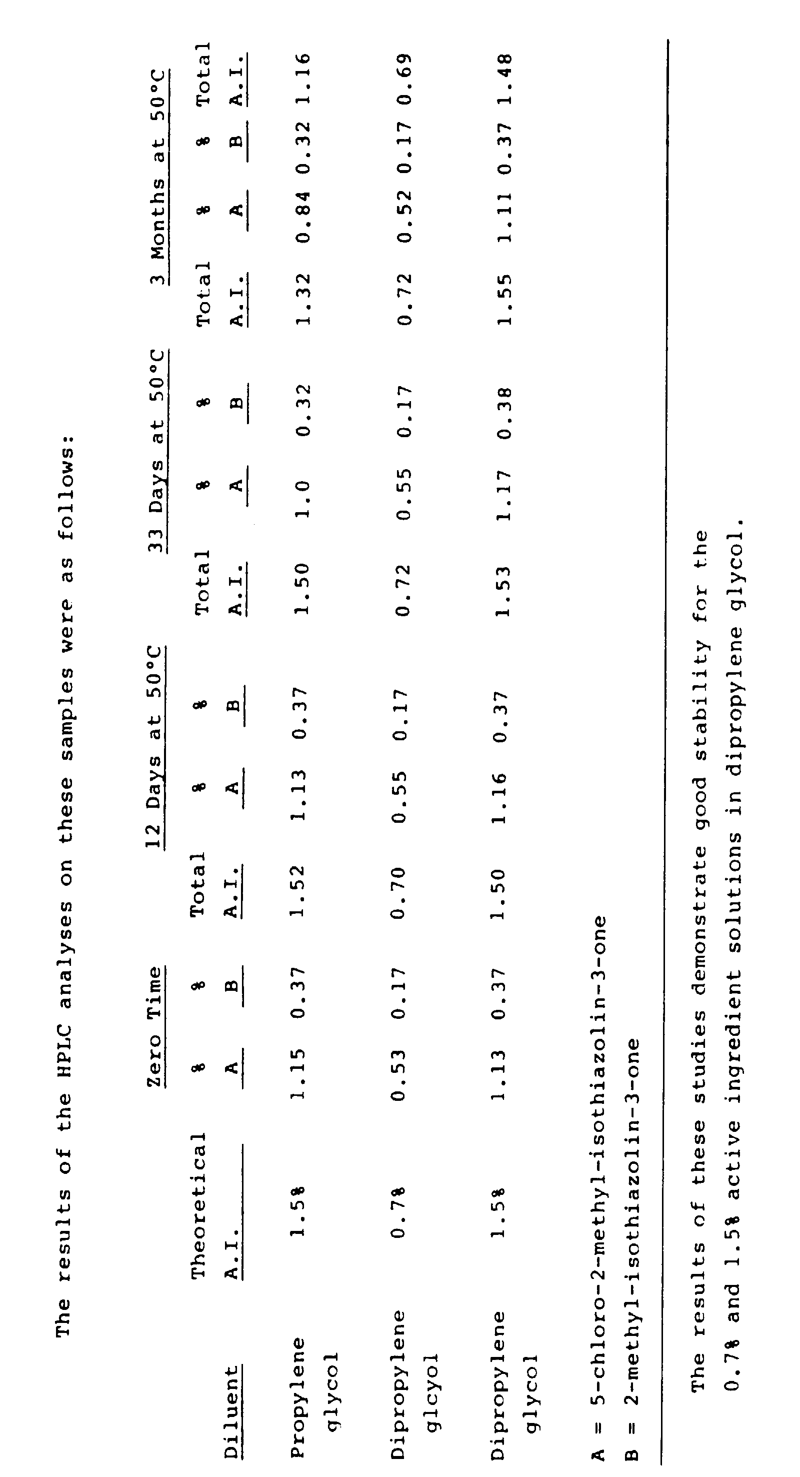

### Example 6

### Additional Thermal Stability Tests

Additional thermal stability tests were initiated on 0.7% and 1.5% 5-chloro-2-methyl-4-isothiazolin-3-one solutions in both propylene glycol and dipropylene glycol. The solutions were prepared by diluting KATHON biocide (see footnote, Example 4) to 1.5% active ingredient with the indicated solvents (w/w).

One hundred gram aliquots of each of the four solutions were placed in both 55°C and 75°C hot air ovens. Three gram aliquots were removed from each of the samples just prior to placement in the ovens, and again after 2,4,6 and 8 weeks at the indicated temperatures. The three gram aliquots were analyzed by HPLC for 5-chloro-2-methyl-4-isothiazolin-3-one content at the time they were removed from the stored samples.

The results of the HPLC analyses of these samples were as follows:

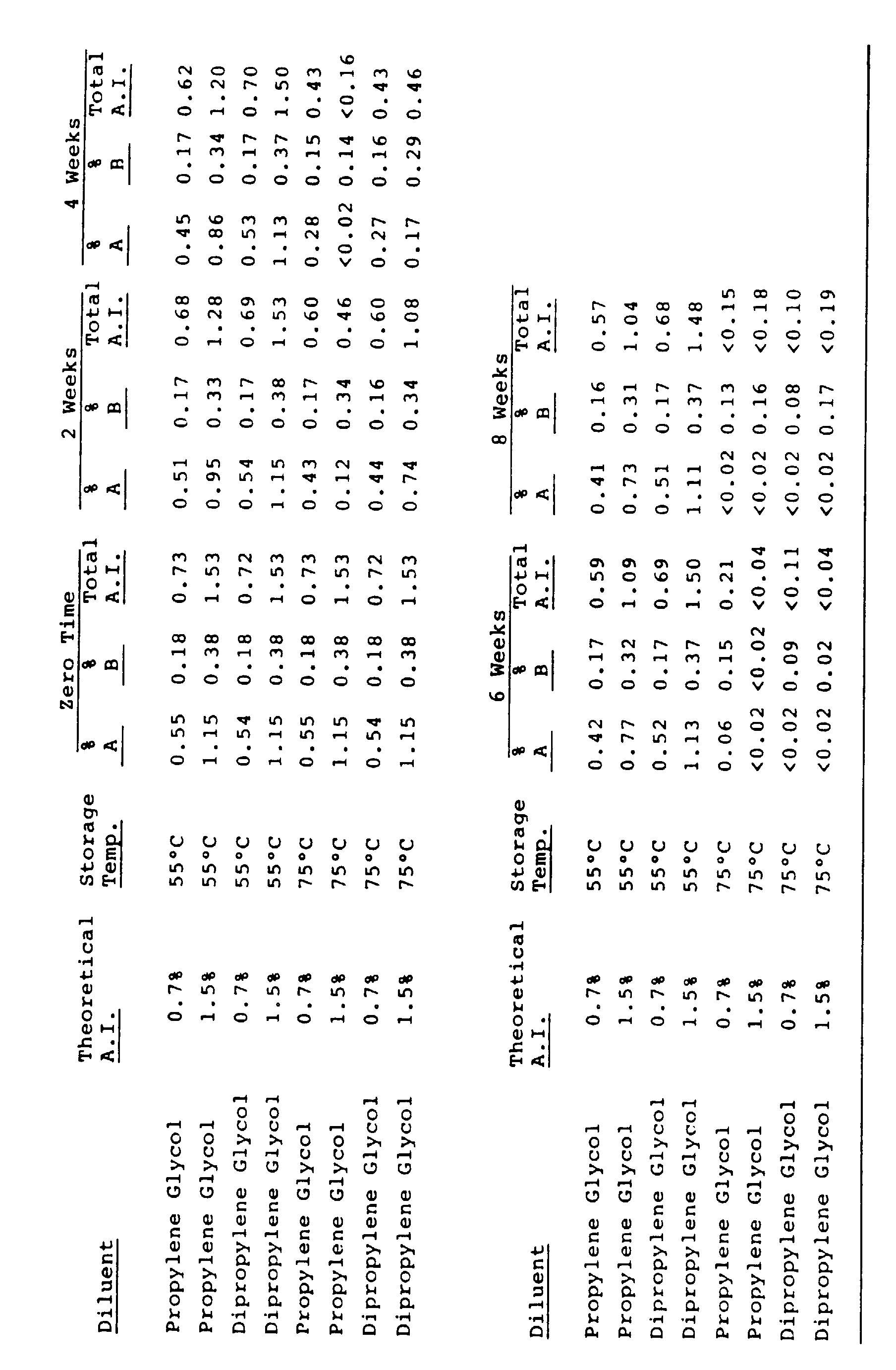

### Example 7

### Addition of N-Methyl-5-chloroisothiazolin-3-one To Salt-sensitive Emulsions.

### Gum Formation During the Addition of N-methyl-5-chloroisothiazolin-3-one to an Unstirred Aqueous Latex

A 3% Al, 2% NaCI solution in dipropylene glycol is diluted 50:50 by weight with water to give solution *A* containing 1.5% Al and 1% NaCI. An aqueous solution containing 15% Al N-methyl-5-chloroisothiazolin-3-one, 15% Mg(NO₃)₂ as a stabilization salt, and 9% MgCl₂ as a neutralization salt is diluted 10-fold with water to give solution *B* containing 1.5% Al, 0.9% MgCl₂, and 1.5% Mg(NO₃)₂ (the 15% Al concentrate simulates Kathon biocide - see footnote in example 4). Where these solutions are added to the unstirred emulsion (to provide 20 ppm Al), and allowed to stand several minutes and then filtered through a 100-mesh screen, it will be seen that no gum will form using solution *A,* while gum (>100 mg) will form using solution *B*.

## Claims

1. The use of 68-98.85% by weight of one or more hydroxy solvents of the formulae: 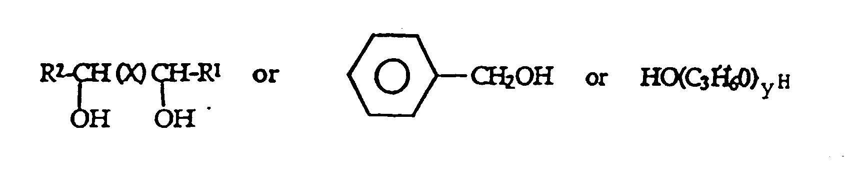 wherein R² and R¹ are (C₁-C₆) alkyl, X is -CH₂OCH₂- or (CH₂)ₙ where n is an integer of 1 to 4, and y is an integer of 1 to 150; as a complete replacement for nitrate stabilisation salt in a stabilized isothiazolone solution which comprises from 0.5% to 7% isothiazolone of the formula: 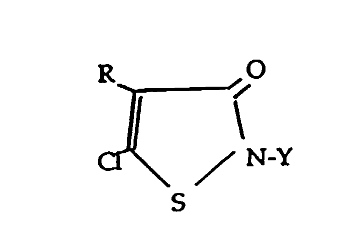 wherein
Y is an alkyl group of 1 to 8 carbon atoms optionally substituted with one or more hydroxy, halo, cyano, alkylamino, dialkylamino, arylamino, carboxy, carbalkoxy, alkoxy, aryloxy, alkylthio, arylthio, isothiazolonyl, haloalkoxy, carbamoxy, morpholino, piperidino or pyrrolidino groups, an unsubstituted or halo-substituted alkenyl or alkynyl of 2 to 8 carbon atoms, an unsubstituted cycloalkyl of 5 to 8 carbon atoms, an aralkyl group, optionally substituted with one or more halo, nitro, (C₁-C₄)alkyl or (C₁-C₄)alkoxy groups, or an aryl group optionally substituted with one or more halo, cyano, nitro, (C₁-C₄)alkyl, (C₁-C₄)alkoxy C₁-C₄ alkyl-acylamino, (C₁-C₄)carbalkoxy or sulfamyl groups; R is hydrogen, halo, or (C₁-C₄)alkyl group; from 0.5% to 14% water, from 0.15 to 7% neutralisation salt, and no stabilisation salt, the percentages being by weight of the four components.

2. Use according to Claim 1 wherein the isothiazolone content is from 1 to 2% by weight, the water content is from 1 to 4% by weight, the solvent content is from 92 to 97.7% by weight, and the neutralisation salt content is from 0.3 to 2% by weight.

3. Use according to any preceding Claim wherein the solvent comprises one or more of propylene glycol, dipropylene glycol, polypropylene glycol, 1,5-pentanediol or benzyl alcohol.

4. Use according to any preceding Claim wherein the isothiazolone comprises 5-chloro-2-methyl isothiazolin-3-one.

5. Use according to any preceding Claim wherein the neutralisation salt comprises copper chloride, sodium chloride and/or magnesium chloride.

## Patentansprüche

1. Verwendung von 68-98,85 Gew.-% eines oder mehrerer Hydroxylösemittel der Formeln 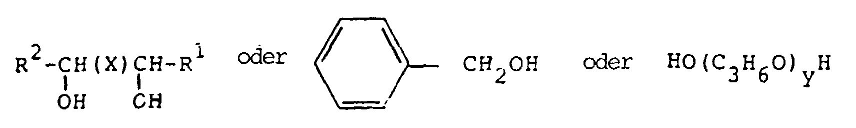 worin R² und R¹ (C₁-C₆)-Alkyl sind, X -CH₂OCH₂- oder -(CH₂)ₙ- ist, wobei n eine ganze Zahl von 1 bis 4 ist und y eine ganze Zahl von 1 bis 150 ist, als vollständigen Ersatz von Nitratsalzstabilisator in einer stabilisierten Isothiazolonlösung, enthaltend 0,5-7% Isothiazolon der Formel 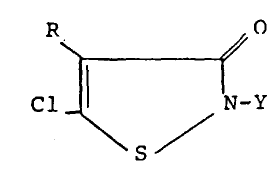 in der Y eine Alkylgruppe mit 1 bis 8 Kohlenstoffatomen, gegebenenfalls substituiert mit einer oder mehreren Hydroxy-, Halogen-, Cyano-, Alkylamino-, Dialkyl amino-, Arylamino-, Carboxy-, Carbalkoxy-, Alkoxy-, Aryloxy-, Alkylthio-, Arylthio-, Isothiazolonyl-, Halogenalkoxy-, Carbamoxy-, Morpholino-, Piperidino- oder Pyrrolidinogruppen, eine nichtsubstituierte oder mit Halogen substituierte Alkenyl- oder Alkynylgruppe mit 2 bis 8 Kohlenstoffatomen, eine nichtsubstituierte Cycloalkylgruppe mit 5 bis 8 Kohlenstoffatomen, eine Aral kyl gruppe, gegebenenfalls substituiert mit einer oder mehreren Halogen-, Nitro-, (C₁-C₄)-Alkyl- oder (C₁-C₄)-Alkoxygruppen, oder eine Arylgruppe, gegebenenfalls substituiert mit einer oder mehreren Halogen-, Cyano-, Nitro-, (C₁-C₄)-Alkyl-, (C₁-C₄)-Alkoxy-, (C₁-C₄)-Alkylacylamino-, (C₁-C₄)-Carbalkoxy- oder Sulfamylgruppen, ist, R Wasserstoff, Halogen oder eine (C₁-C₄)-Alkylgruppe ist, 0,5-14% Wasser, 0,15-7% Neutralisationssalz und kein Stabilisierungssalz, wobei sich die Prozentsätze auf das Gewicht der 4 Komponenten beziehen.

2. Verwendung nach Anspruch 1, dadurch gekennzeichnet, daß der Isothiazolongehalt 1.2 Gew.-%, der Wassergehalt 1-4 Gew.-%, der Lösemittelgehalt 92-97,7 Gew.-% und der Neutralisationssalzgehalt 0,3-2 Gew.-% beträgt.

3. Verwendung nach einem der vorstehenden Ansprüche, dadurch gekennzeichnet, daß das Lösemittel eines oder mehrere von Propylenglykol, Dipropylenglykol, Polypropylenglykol, 1,5-Pentandiol oder Benzylalkohol ist.

4. Verwendung nach einem der vorstehenden Ansprüche, dadurch gekennzeichnet, daß das Isothiazolon 5-Chlor-2-methylisothiazolin-3-on ist.

5. Verwendung nach einem der vorstehenden Ansprüche, dadurch gekennzeichnet, daß das Neutralisationssalz Kupferchlorid, Natriumchlorid und/oder Magnesiumchlorid ist.

## Revendications

1. Utilisation de 68 à 98,85 % en poids d'un ou de plusieurs solvants hydroxylés de formules : 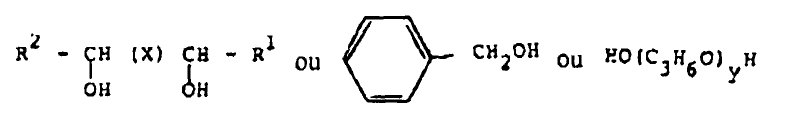 où R² et R¹ sont un hydrogène ou un alcoyl inférieur, X est -CH₂OCH₂- ou -(CH₂)ₙ- où n est un entier de 1 à 4, et y est un entier de 1 à 150; en remplacement total d'un sel de stabilisation de type nitrate dans une solution stabilisée d'isothiazolone qui comprend 0,5 % à 7 % d'izothiazolone de formule : 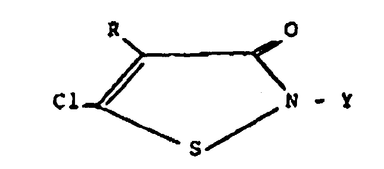 dans laquelle
Y est un groupe alcoyle de 1 à 8 atomes de carbone facultativement substitué par un ou plusieurs groupes hydroxy, halogéno, cyano, alcoylamino, dialcoylamino, arylamino, carboxy, carbalcoxy, alcoxy, aryloxy, alcoylthio, arylthio, isothiazolonyles, halogénoalcoxy, carbamoxy, morpholino, pipéridino ou pyrrolidino, un alcényle ou alcynyle de 2 à 8 atomes de carbone non substitués ou halogéno-substitués, un cycloalcoyle non substitué de 5 à 8 atomes de carbone, un groupe aralcoyle, facultativement substitué par un ou plusieurs groupes halogéno, nitro, alcoyles en C₁-C₄ ou alcoxy en C₁-C₄, ou un groupe aryle facultativement substitué par un ou plusieurs groupes halogéno, cyano, nitro, alcoyles en C₁-C₄, alcoxy en C₁-C₄(alcoyl en C₁-C₄)acylamino, carbalcoxy en C₁-C₄ ou sulfamyles ;
R est un hydrogène, un halogéno ou un alcoyle en C₁-C₄ ; 0,5 % à 14 % d'eau, 0,15 à 7 % de sel de neutralisation, et pas de sel de stabilisation, les pourcentages étant rapportés au poids des quatre composants.

2. Utilisation selon la revendication 1, dans laquelle la teneur en isothiazolone est de 1 à 2 % en poids, la teneur en eau est de 1 à 4 % en poids, la teneur en solvant est de 92 à 97,7 % en poids et la teneur en sel de neutralisation est de 0,3 à 2 % en poids.

3. Utilisation selon l'une quelconque des revendications précédentes, dans laquelle le solvant comprend un ou plusieurs composés parmi le propylèneglycol, le dipropylèneglycol, le polypropylèneglycol, le 1,5-pentanediol ou l'alcool benzylique.

4. Utilisation selon l'une quelconque des revendications précédentes, dans laquelle l'isothiazolone comprend la 5-chloro-2-méthylisothiazoline-3-one.

5. Utilisation selon l'une quelconque des revendications précédentes, dans laquelle le sel de neutralisation comprend du chlorure de cuivre, du chlorure de sodium et/ou du chlorure de magnésium.
